(19) Europäisches Patentamt
European Patent Office
Office européen des brevets

(11) Veröffentlichungsnummer: **0 576 906 A1**

(12) **EUROPÄISCHE PATENTANMELDUNG**

(21) Anmeldenummer: **93109574.9**

(22) Anmeldetag: **16.06.93**

(51) Int. Cl.⁵: **C07D 513/04**, A61K 31/54, //(C07D513/04,333:00,279:00)

(30) Priorität: **03.07.92 AT 1360/92**

(43) Veröffentlichungstag der Anmeldung: **05.01.94 Patentblatt 94/01**

(84) Benannte Vertragsstaaten: **AT BE CH DE DK ES FR GB GR IE IT LI LU NL PT SE**

(71) Anmelder: **CHEMISCH PHARMAZEUTISCHE FORSCHUNGS-GESELLSCHAFT m.b.H.**
**St. Peter-Strasse 25**
**A-4021 Linz(AT)**

(72) Erfinder: **Binder, Dieter, Dr.**
**Sieveringerstrasse 207**
**A-1190 Wien(AT)**
Erfinder: **Weinberger, Josef, Dr.**
**Bahnhofstrasse 5**
**A-4540 Bad Hall(AT)**

(74) Vertreter: **Kunz, Ekkehard, Dr.**
**Chemie Linz Ges.m.b.H.**
**Abteilung Patente**
**St. Peter-Str. 25**
**A-4021 Linz (AT)**

(54) **Neue Thienothiazinderivate, Verfahren zu ihrer Herstellung und ihre Verwendung als Antiinflammatorium und Analgeticum.**

(57) Die Erfindung betrifft Thienothiazin-Derivate der allgemeinen Formel

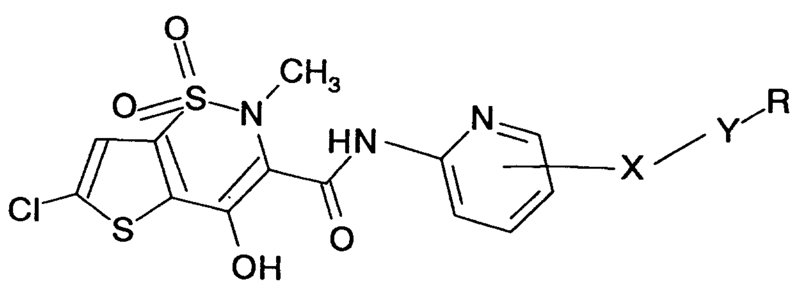

I

in der
X eine Einfachbindung, eine geradkettige oder verzweigte Kohlenstoffkette mit 1 - 12 C-Atomen in der Kette, wobei diese Kette eine oder mehrere Doppel- oder Dreifachbindungen und/oder ein oder mehrere Heteroatome enthalten kann,
Y eine Einfachbindung,
und
R Wasserstoff, einen monocyclischen oder polycyclischen, gegebenenfalls teilweise hydrierten Aryl-, Heteroaryl-, Aryloxy-, Heteroaryloxy-, Arylaza-, Heteroarylaza-, Arylthio-, oder Heteroarylthiorest, der gegebenenfalls durch Niederalkyl, ein- oder mehrfach halogeniertes Niederalkyl, perfluoriertes Niederalkyl, Alkoxy oder Halogen substituiert sein kann, mit der Maßgabe, daß R nicht Wasserstoff bedeutet, wenn X und Y eine Einfachbindung bedeuten,
bedeutet, ein Verfahren zu deren Herstellung und deren Verwendung

EP 0 576 906 A1

Die Erfindung betrifft neue, therapeutisch wertvolle Thienothiazinderivate.

Aus US 4,180,662 sind Thienothiazinderivate bekannt, die eine Cyclooxygenasehemmung bewirken. Diese Verbindungen sind am Pyridinkem unsubstituiert.

Es konnten nun neue, am Pyridinkern substituierte Thienothiazinderivate gefunden werden, die bei weitgehender Beibehaltung der Cyclooxygenasehemmung eine signifikant erhöhte Hemmung der 5-Lipoxigenase bewirken.

Gegenstand der Erfindung sind demnach Thienothiazindenvate der allgemeinen Formel

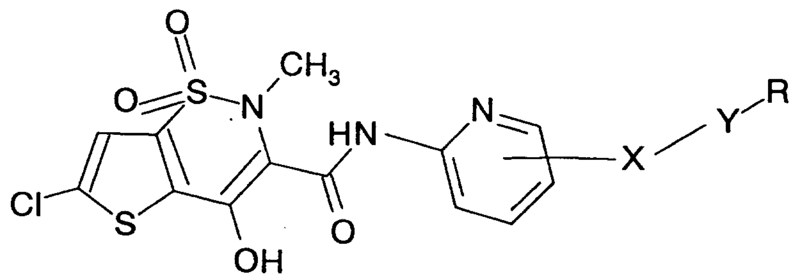

I

in der

X eine Einfachbindung, eine geradkettige oder verzweigte Kohlenstoffkette mit 1 - 12 C-Atomen in der Kette, wobei diese Kette eine oder mehrere Doppel- oder Dreifachbindungen und/oder ein oder mehrere Heteroatome enthalten kann,

Y eine Einfachbindung,

und

R Wasserstoff, einen monocyclischen oder polycyclischen, gegebenenfalls teilweise hydrierten Aryl-, Heteroaryl, Aryloxy-, Heteroaryloxy-, Arylaza-, Heteroarylaza, Arylthio-, oder Heteroarylthiorest, der gegebenenfalls durch Niederalkyl, ein- oder mehrfach halogeniertes Niederalkyl, perfluoriertes Niederalkyl, Alkoxy oder Halogen substituiert sein kann, mit der Maßgabe, daß R nicht Wasserstoff bedeutet, wenn X und Y eine Einfachbindung bedeuten,

bedeutet.

Bevorzugt sind solche Verbindungen, in denen der Substituent R an der 6-Position des Pyridins verknüpft ist.

Niederalkyl bedeutet einen Alkylrest mit 1 - 4 C-Atomen, beispielsweise einen Methyl-, einen Ethyl-, einen Propyl-, einen i-Propyl-, einen Butyl-, einen i-Butyl-, einen t-Butylrest.

Der Begriff Heteroatome umfaßt O, S, N.

Als mono- oder polycyclische, gegebenenfalls teilweise hydrierte Aryl- oder Heteroarylreste kommen 5-12 gliedrige, gegebenenfalls teilweise hydrierte aromatische oder heteroaromatische Reste in Frage, wie beispielsweise ein Phenyl-, Thienyl-, Furyl-, Pyrrolyl-, Pyridinyl-, Pyrimidinyl-, Pyranyl- Thiadiazinyl-, Azepinyl, oder Benzofuryl- oder Chinolinylrest.

Ein weiterer Gegenstand der Erfindung ist ein Verfahren zur Herstellung von Verbindungen der allgemeinen Formel I, das dadurch gekennzeichnet ist, daß man eine Verbindung der allgemeinen Formel II

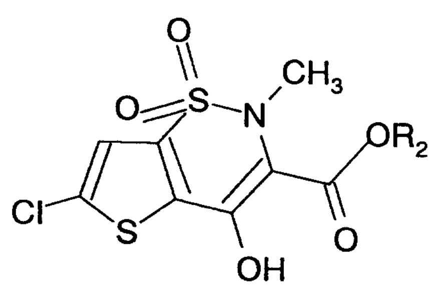

II

in der $R_2$ eine geradkettige oder verzweigte Alkylgruppe mit 1 - 4 C-Atomen bedeutet mit einer Verbindung der allgemeinen Formel III

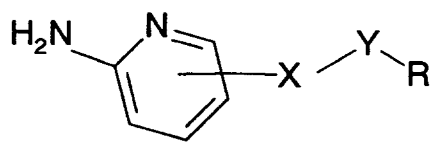

III

in der X, Y, R die oben genannte Bedeutung haben, umsetzt und die so erhaltenen Verbindungen der allgemeinen Formel (I) gegebenenfalls in ihre pharmazeutisch verwendbaren Salze überführt.

Das erfindungsgemäße Verfahren kann vorzugsweise auf eine der folgenden Weisen durchgeführt werden,indem man

a) eine Verbindung der allgemeinen Formel II in einem inerten Lösungsmittel, wie Diethylether, Dioxan, Toluol, Benzol oder dergleichen, löst und bei einer Temperatur zwischen - 20 °C und 100 °C ein Äquivalent einer starken Base, wie Butyllithium oder LDA, in einem inerten Lösungsmittel, beispielsweise n-Hexan, gegebenenfalls unter Inertgas zusetzt, zu dieser Salzlösung 1-10 Äquivalente, vorzugsweise 1-5 Äquivalente einer Verbindung der allgemeinen Formel III zusetzt, mindestens 1 Äquivalent der starken Base zusetzt und zwischen 0,5 und 60 Stunden, vorzugsweise 1-48 Stunden bei -20 °C bis 100 °C, vorzugsweise 0 °C bis 70 °C rührt, oder

b) die Verbindungen der allgemeinen Formel II und der allgemeinen Formel III in einem inerten hochsiedenendne Lösungsmittel, wie Toluol, Xylol, Pyridin, Chinolin, Dimethylformamid, Dimethylsulfoxid oder Hexamethylphosphorsäuretriamid löst und dieses Gemisch 1-30 Stunden auf 100 °C bis 200 °C erhitzt.

Die bei dieser Umsetzung erhaltenen Verbindungen der allgemeinen Formel (I) sind saure Verbindungen und können auf übliche Weise mit anorganischen oder organischen Basen in ihre pharmazeutisch verträglichen Salze übergeführt werden.

Die Salzbildung kann beispielsweise durchgeführt werden, indem man die Verbindungen der Formel (I) in einem geeigneten Lösungsmittel, z.B. Wasser, einem niederen aliphatischen Alkohol, Tetrahydrofuran, Dioxan, Benzol, Diethylether, Dimethylformamid oder Dimethylsulfoxid löst, eine äquivalente Menge der gewünschten Base zusetzt, für eine gute Durchmischung sorgt und nach beendeter Salzbildung das Lösungsmittel im Vakuum abzieht. Gegebenenfalls können die Salze nach ihrer Isolierung weiter gereinigt werden, beispielsweise durch umkristallisieren.

Pharmazeutisch verträgliche Salze sind beispielsweise Metallsalze, insbesondere Alkalimetallsalze und Erdalkalimetallsalze, wie Natrium-, Magnesium- oder Calciumsalze. Andere pharmazeutische Salze sind beispielsweise leicht kristallisierbare Ammoniumsalze. Letztere leiten sich ab von Ammoniak oder organischen Aminen, wie z.B. Mono-, Di- oder Tri-nieder-(alkyl-, cycloalkyl- oder hydroxyalkyl-)aminen, Niederalkylendiaminen, (Hydroxy-niederalkyl)- oder (Aryl-niederalkyl)niederalkylammoniumbasen, beispielweise Methylamin, Diethylamin, Triethylamin, Dicyclohexylamin, Triethanolamin, Ethylendiamin, Tris(hydroxy-methyl-)aminomethan, Benzyltrimethylammoniumhydroxid und dergleichen.

Die Verbindungen der allgmeinen Formeln (II) und (III) sind literaturbekannt oder können analog dazu nach üblichen und dem Fachmann geläufige Methoden hergestellt werden.

Die erfindungsgemäßen Verbindungen der allgemeinen Formel (I) und ihre Salze sind oral wirksam und zeigen im Vergleich zu am Pyridinkem unsubstituierten Verbindungen, wie beispielsweise dem aus US 4,180,662 bekannten 6-Chlor-4-hydroxy-2-methyl-N-(2-pyridyl)-2H-thieno(2,3-e)1,2-thiazin-3-carboxamid-1,1-dioxid ("Lornixicam") überraschender Weise eine signifikant höhere Hemmung der 5-Lipoxigenase bei weitgehendster Beibehaltung der Cyclooxygenasehemmung.

Sie sind daher zur Behandlung von Beschwerden, die durch das natürliche Produkt der 5-Lipoxygenase, nämlich dem Leukotrien-$B_4$, mitverursacht werden, wie z.B. Entzündung und Schmerz bei allergischem Asthma, Arthrits, Hautallergie usw. besonders gut geeignet.

Aufgrund dieser pharmakologischen Eigenschaften können die neuen Verbindungen allein oder in Mischung mit anderen Wirksubstanzen in Form üblicher galenischer Zubereitungen als Heilmittel zur Behandlung von Erkrankungen, die durch die Hemmung der 5-Lipoxygenase geheilt oder gelindert werden, Verwendung finden.

Die Erfindung bezieht sich weiterhin auf Heilmittel, die z.B. in Form pharmazeutischer Präparate Verwendung finden, welche die erfindungsgemäßen Verbindungen der allgemeinen Formel (I) und ihre Salze in Mischung mit einem für die orale, enterale, parenterale und topicale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline

und dergleichen enthalten.

Die pharmazeutischen Präparate können in fester Form, z.B. als Tabletten, Filmtabletten, Dragees, Suppositorien, Kapseln, Mikrokapseln oder in flüssiger Form, z.B. als Lösungen, Injektionslösungen, Suspensionen oder Emulsionen oder in Zusammensetzungen mit verzögerter Freigabe des Wirkstoffes vorliegen.

Gegebenenfalls können sie sterilisiert werden und/oder sie enthalten Hilfsstoffe wie Konservierungs-, Stabilisierungs- oder Emulgiermittel, Salze zur Veränderung des osmotischen Drucks oder Puffer.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen zusammen mit den oben genannten Hilfs- und/oder Trägerstoffen zu Kombinationspräparaten formuliert werden.

Die neuen Verbindungen können in den erfindungsgemäßen Zusammensetzungen in einem Anteil von 4-200 mg pro Tablette vorhanden sein, wobei der Rest ein pharmazeutisch annehmbarer Füllstoff ist.

Eine geeignete Dosis zur Verabreichung der Verbindungen beträgt etwa 4-200 mg/kg pro Tag, jedoch kommen, je nach Zustand des zu behandelnden Patienten auch andere Dosen in Frage. Die neuen Verbindungen können in mehren Dosen und auf oralem Weg verabreicht werden.

Beispiel 1:

6-Chlor-4-hydroxy-2-methyl-N-[3-(2-phenylethyl)-2-pyridyl)]-2H-thieno[2,3-e]1,2-thiazin-3-carboxamid-1,1-dioxid

5.50 g (7.8 mMol) 6-Chlor-4-hydroxy-2-methyl-2H-thieno(2,3-e)1,2-thiazin-3-carbonsäuremethylester und 7.04 g (35.5 mMol) 3-(2-Phenylethyl)-pyridin-2-amin werden in 150 ml abs. Xylol gelöst und 5 Stunden zum Sieden erhitzt. Dabei werden insgesamt 40 ml Flüssigkeit abdestilliert und laufend durch frisches Lösungsmittel ersetzt. Das abgekühlte Reaktionsgemisch wird filtriert, das erhaltene Kristallisat mit Methylenchlorid und Methanol digeriert aus 150 ml DMF umkristallisiert und im Vakuum bei 50 °C getrocknet.
Ausbeute: 5.34 g gelbe Kristalle (63% der Theorie)
Fp.: 251 - 252 °C (DMF, Zersetzung)
$^1$H-NMR: (CF3COOD)
δ (ppm): 7.97 (d, 1H, Py-H6*); 7.86 (d, 1H, Py-H4*); 7,22 (dd, 1H, Py-H5); 6.96 (s, 1H, Th-H); 6.93-6.62 (m, 5H, Bz-H2-6); 2.90-2.55 (m, 4H, C<u>H2</u>-C<u>H2</u>); 2.61 (s, 3H, N-C<u>H3</u>)
$^{13}$C-NMR: (CF3COOD)
δ (ppm): 171.5; 161.7: 151.8; 148.4; 145.5; 141.9; 139.4; 135.0; 133.4; 132.5; 131.6; 130.5; 126.5; 125.4; 112.7; 43.1; 37.8; 34.8

Beispiel 2

6-Chlor-4-hydroxy-2-methyl-N-[4-(2-phenylethyl)-2-pyridyl]-2H-thieno[2,3-e]1,2-thiazin-3-carboxamid-1,1-dioxid

0.96 g (3.10 mMol) 6-Chlor-4-hydroxy-2-methyl-2H-thieno[2,3-e]1,2-thiazin-3-carbonsäuremethylester werden unter trockener Stickstoffatmosphäre in 21 ml abs. Toluol gelöst und bei 2 °C mit 1.24 ml (3.10 mMol) einer 2,5m Lösung von Butyllithium in n-Hexan versetzt. Anschließend werden 1.23 g (6.20 mMol) 4-(2-Phenylethyl)-pyridin-2-amin in 13 ml abs. Toluol in 15 Minuten so zugetropft, daß die Temperatur 25 °C nicht übersteigt, alle Substanzen durch ca. 15 minütiges Erwärmen auf 50 °C gelöst und innerhalb von 20 Minuten 2.48 ml (6.20 mMol) einer 2,5m Lösung von Butyllithium in n-Hexan bei einer Temperatur von 20-35 °C zugetropft.

Das Reaktionsgemisch wird 2 Stunden bei Raumtemperatur, 1.5 Stunden bei 55 °C und über Nacht bei Raumtemperatur gerührt, mit 0.37 g (6.20 mMol) Eisessig versetzt, 1 Stunde bei 1 °C mit 40 ml Wasser gerührt, die ausgefällten Kristalle abfiltriert und mit kaltem Wasser gewaschen. Das erhaltene Lithiumsalz wird nun mit 50 ml 2n HCl hydrolysiert, die wäßrige Suspension dreimal mit je 50 ml heißem Toluol extrahiert und die organischen Extrakte eingedampft. Die erhaltenen Kristalle werden aus ca. 40 ml Benzol umkristallisiert und bei 100 °C im Vakuum getrocknet.
Ausbeute: 1.01 g gelbe Kristalle (68% d.Th.)
Fp.: 214-215 °C (Benzol, Zers.)
$^1$H-NMR: (DMSO)
δ (ppm): 8.17 (d, 1H, Py-H6); 7.57 (s, 2H, Th-H, Py-H3); 7.35-7.12 (m, 6H, Py-H5, Bz-H2-6); 3.13-2.90 (m,

4H, CH2-CH2); 2.90 (s, 3H, N-CH3)
$^{13}$C-NMR: (DMSO)
δ (ppm): 165.0; 164.2; 161.2; 148.6; 143.2; 140.2; 137.1; 137.0; 134.3; 128.3; 126.1; 122.6; 118.6; 114.9; 107.6; 39.1; 36.6; 34.7

Beispiel 3

6-Chlor-4-hydroxy-2-methyl-N-[5-(2-phenylethyl)-2-pyridyl]-2H-thieno[2,3-e]1,2-thiazin-3-carboxamid-1,1-dioxid

1.93 g (6.2 mMol) 6-Chlor-4-hydroxy-2-methyl-2H-thieno[2,3-e]1,2-thiazin-3-carbonsäuremethylester werden unter trockener Stickstoffatmosphäre in 42 ml abs. Toluol gelöst und bei 2°C mit 2.5 ml (6.2 mMol) einer 2,5m Lösung von Butyllithium in n-Hexan versetzt. Anschließend werden 2.47 g (6.20 mM) 5-(2-Phenylethyl)-pyridin-2-amin in 23 ml abs. Toluol in 15 Minuten bei 20-25°C zugetropft, ca 15 Minuten bei Raumtemperatur gerührt und innerhalb von 20 Minuten 5.0 ml (12.5 mMol) einer 2,5m Lösung von Butyllithium in n-Hexan bei einer Temperatur von 20-35°C zugetropft.

Das Reaktionsgemisch wird nun über Nacht bei Raumtemperatur gerührt, mit 100 ml 2n HCl 30 Minuten gerührt, die ausgefällten Kristalle abfiltriert und mit kaltem Wasser gewaschen. Die wäßrige Phase wird noch 12 Stunden auf 0°C gekühlt und das restliche auskristallisierte Produkt abfiltriert und mit kaltem Wasser gewaschen. Die vereinigten Fraktionen werden bei 50°C im Vakuum über Phosphorpentoxid getrocknet, aus 170 ml Ethylenglycolmonomethylether umkristallisiert und bei 100°C im Vakuum getrocknet.
Ausbeute: 1.89 g gelbe Kristalle (64% d.Th.)
Fp.: 224-227°C (Ethylenglycolmonomethylether, Zers.)
$^{1}$H-NMR: (DMSO)
δ (ppm); 8.21-8.03 (m, 2H, Py-H3,6); 7.69 (d, 1H, Py-H4); 7.57 (s, 1H, Th-H), 7.37-7.12 (m, 5H, Bz-H2-6); 3.03-2.78 (m, 7H, CH2-CH2, N-CH3)
$^{13}$C-NMR: (CF3COOD)
δ (ppm): 172.6; 161.8; 152.3; 149.2; 145.7; 142.4; 141.6; 140.7; 139.5; 135.5; 132.2; 132.0; 130.2; 126.4; 120.0; 112.7; 43.1; 39.5; 37.0

Beispiel 4

6-Chlor-4-hydroxy-2-methyl-N-[6-(2-phenylethyl)-2-pyridyl]-2H-thieno[2,3-e]1,2-thiazin-3-carboxamid-1,1-dioxid

1.43 g (4.61 mMol) 6-Chlor-4-hydroxy-2-methyl-2H-thieno[2,3-e]1,2-thiazin-3-carbonsäuremethylester werden unter trockener Stickstoffatmosphäre in 34 ml abs. Toluol gelöst und bei 2°C mit 1.85 ml (4.61 mMol) einer 2,5m Lösung von Butyllithium in n-Hexan versetzt. Anschließend werden 1.83 g (9.23 mMol) 6-(2-Phenylethyl)-pyridin-2-amin in 17 ml abs. Toluol in 20 Minuten so zugetropft, daß die Temperatur 25°C nicht übersteigt, 15 Minuten bei Raumtemperatur gerührt und innerhalb von 20 Minuten 3.70 ml (9.23 mMol) einer 2,5m Lösung von Butyllithium in n-Hexan bei einer Temperatur von 20-35°C zugetropft.
Das Reaktionsgemisch wird nun 2 Stunden auf 70°C erwärmt, abgekühlt, mit 0.55 g (9.23 mMol) Eisessig versetzt, 1 Stunde bei 0°C gerührt und die ausgefällten Kristalle abfiltriert und mit kaltem Wasser gewaschen. Das erhaltene Lithiumsalz wird nun mit 75 ml 2n HCl hydrolysiert, die wäßrige Suspension zweimal mit je 75 ml heißem Toluol extrahiert und die organischen Extrakte eingedampft. Die erhaltenen Kristalle werden aus ca. 70 ml Acetonitril umkristallisiert und bei 100°C im Vakuum getrocknet.
Ausbeute: 1.33 g gelbe Kristalle (61% d.Th.)
Fp.: 206-208°C (Acetonitril, Zers.)
$^{1}$H-NMR: (CDCl3)
δ (ppm): 7.37-7.11 (m, 7H, Bz-H2-6, Py-H3-4); 6.90 (d, 1H, Py-H5); 3.17-2.93 (m, 7H, CH2-CH2, N-CH3)
$^{13}$C-NMR: (DMSO)
δ (ppm): 164.9; 162.3; 153.4; 149.6; 143.9; 142.2; 140.0; 137.0; 134.5; 128.3; 126.2; 122.6; 117.6; 113.3; 108.3; 39.2; 35.3; 34.2
Auf analoge Weise wurden folgende Verbindungen hergestellt

| Bsp | Verbindung | Fp (°C) |
|---|---|---|
| 5 | 6-Chlor-4-hydroxy-2-methyl-N-[6-(2,4-difluorphenyloxy-methyl)]-2-pyridyl-2H-thieno[2,3-e]-1,2-thiazin-3-carbox-amid-1,1,-dioxid | 174-177 |
| 6 | 6-Chlor-4-hydroxy-2-methyl-N-[6-(2-furyl)]-2-pyridyl-2H-thieno[2,3-e]-1,2-thiazin-3-carbox-amid-1,1,-dioxid | 207-209 (Zers.) |
| 7 | 6-Chlor-4-hydroxy-2-methyl-N-[6-phenylethinyl]-2-pyridyl-2H-thieno 2,3-e -1,2-thiazin-3-carbox-amid-1,1,-dioxid | 222-229 (Zers.) |
| 8 | 6-Chlor-4-hydroxy-2-methyl-N-[6-(3-phenylpropyl)]-2-pyridyl-2H-thieno[2,3]-e -1,2-thiazin-3-carbox-amid-1,1,-dioxid | 159-162 |
| 9 | 6-Chlor-4-hydroxy-2-methyl-N-[6-(4-phenylbutyl)]-2-pyridyl-2H-thieno[2,3-e]-1,2-thiazin-3-carbox-amid-1,1,-dioxid | 130-138 (Zers.) |
| 10 | 6-Chlor-4-hydroxy-2-methyl-N-[6-(4-fluorphenoxy- | |

| | | |
|---|---|---|
| | methyl]-2-pyridyl-2H-thieno 2,3-e -1,2-thiazin-3-carboxamid-1,1,-dioxid | 172-174 (Zers.) |
| 11 | 6-Chlor-4-hydroxy-2-methyl-N-[6-(4-phenoxymethyl)]-2-pyridyl-2H-thieno[2,3-e]-1,2-thiazin-3-carboxamid-1,1,-dioxid | 169-172 (Zers.) |
| 12 | 6-Chlor-4-hydroxy-2-methyl-N-[6-(2-benzo(b)furyl)]-2-pyridyl-2H-thieno[2,3-e]-1,2-thiazin-3-carboxamid-1,1,-dioxid | 254-256 (Zers.) |
| 13 | 6-Chlor-4-hydroxy-2-methyl-N-[6-phenylmethoxy]-2-pyridyl-2H-thieno[2,3-e]-1,2-thiazin-3-carboxamid-1,1,-dioxid | ab 176 (Zers.) |
| 14 | 6-Chlor-4-hydroxy-2-methyl-N-[6-((E)-2-phenylethenyl)]-2-pyridyl-2H-thieno[2,3-e]-1,2-thiazin-3-carboxamid-1,1,-dioxid | 226-229 (Zers.) |
| 15 | 6-Chlor-4-hydroxy-2-methyl-N-[6-phenylmethyl]-2-pyridyl-2H-thieno[2,3-e]-1,2-thiazin-3-carboxamid-1,1,-dioxid | 176-180 (Zers.) |
| 16 | 6-Chlor-4-hydroxy-2-methyl-N-[6-phenyl]-2-pyridyl-2H-thieno[2,3-e]-1,2-thiazin-3-carboxamid-1,1,-dioxid | 207-208 (Zers.) |
| 17 | 6-Chlor-4-hydroxy-2-methyl-N-[6-propyl]-2-pyridyl-2H-thieno[2,3-e]-1,2-thiazin-3-carboxamid-1,1,-dioxid | 226-230 (Zers.) |
| 18 | 6-Chlor-4-hydroxy-2-methyl-N-[6-ethyl]-2-pyridyl-2H-thieno[2,3-e]-1,2-thiazin-3-carboxamid-1,1,-dioxid | 212-217 (Zers.) |
| 19 | 6-Chlor-4-hydroxy-2-methyl-N-[6-methyl]-2-pyridyl-2H-thieno[2,3-e]-1,2-thiazin-3-carboxamid-1,1,-dioxid | 225-227 (Zers.) |

Beispiel A:

Die Bildung von Prostaglandin D2 durch Neutrophile wurde als Maß für die Cycloxygenase-Aktivität verwendet und die Bildung von Leukotrien B4 als Maß für die 5-Lipoxygenase-Aktivität.

Männlichen Sprague Dawley Ratten (250-300 g) wurde lamda - Caarageenan 1mg intraperitonal (gelöst in 0.5 ml dest. Wasser) injiziert.

Nach 16 Stunden wurden die Ratten durch Exposition in Diethylether getötet. 15 ml eiskalte Hanks balanced salt solution (HBSS) wurden i.p. injiziert, die Neutrophilen durch Absaugen geerntet (10 ml), zentrifugiert (5 min, 100g, 4 °C), die überstehende Lösung dekantiert und die Zellen in HBSS resuspendiert bei 4 °C bis zu einer Konzentration von $5x10^6$ Zellen/ml.

400 µl Zellsuspension (2x106 Zellen), 0,5µl Verbindung gelöst in DMSO und 49,5 µl HBSS wurden 5 min bei 37 °C inkubiert. Dann wurden 50 µl A23187 (2µmol/l Endkonzentration) zugefügt und anschließend

wiederum 5 min bei 37° C inkubiert.
Die Reaktion wurde durch Zentrifugieren 10000g, 3s gestoppt und die überstehende Flüssigkeit in vorgekühlte Plastikröhrchen übergeführt und vor dem Beginn der Radioimmunoassay-Messung im Eisbad max. 1 h belassen.
PGD2 und LTB4 wurde nach Verdünnung mit HBSS mit Hilfe kommerzieller RIA-Kits gemessen.
Als Vergleichssubstanz diente 6-Chlor-4-hydroxy-2-methyl-N-(2-pyridyl)-2H-thieno(2,3-e)1,2-thiazin-3carboxamid-1,1-dioxid ("Lornoxicam", Vbg.A)

| Vbg | IC50 (µmol/l) | |
|---|---|---|
| | PGD2 | LTB4 |
| A | 0.02 | >10 |
| 12 | 0.016 | 2.0 |
| nach Bsp 4 | 0.19 | 2.7 |
| 14 | 0.18 | 4.3 |
| 13 | 0.15 | 5.1 |
| 11 | 0.12 | 7.3 |
| 10 | 0.45 | 4.8 |
| 9 | 0.75 | 1.9 |
| 8 | 0.51 | 4.2 |
| 7 | 0.41 | 3.7 |

Die neuen Verbindungen zeigen zusätzlich zur Hemmung der Cyclooxygenase eine Hemmung der 5-Lipoxygenase, während Verbindung A nur die Cycloxygenase inhibiert. Die neuen Verbindungen bieten daher eine neue, überlegene therapeutische Möglichkeit zur Behandlung von entzündlichen Krankheiten.

## Patentansprüche

1. Thienothiazin-Derivate der allgemeinen Formel

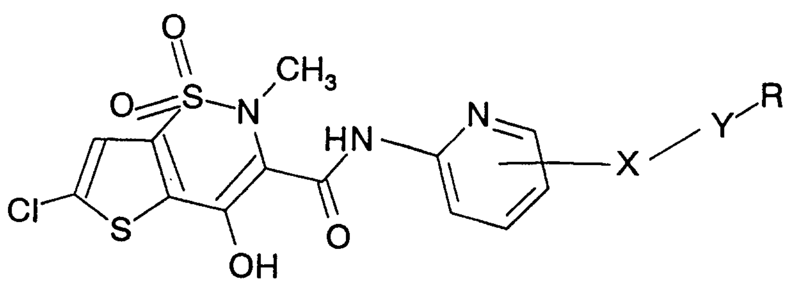

I

in der
X eine Einfachbindung, eine geradkettige oder verzweigte Kohlenstoffkette mit 1 - 12 C-Atomen in der Kette, wobei diese Kette eine oder mehrere Doppel- oder Dreifachbindungen und/oder ein oder mehrere Heteroatome enthalten kann,
Y eine Einfachbindung,
und
R Wasserstoff, einen monocyclischen oder polycyclischen, gegebenenfalls teilweise hydrierten Aryl-, Heteroaryl-, Aryloxy-, Heteroaryloxy-, Arylaza-, Heteroarylaza-, Arylthio-, oder Heteroarylthiorest, der gegebenenfalls durch Niederalkyl, ein- oder mehrfach halogeniertes Niederalkyl, perfluoriertes Niederalkyl, Alkoxy oder Halogen substituiert sein kann, mit der Maßgabe, daß R nicht Wasserstoff bedeutet, wenn X und Y eine Einfachbindung bedeuten,
bedeutet.

2. Verbindungen nach Anspruch 1, in denen die Substituenten an der 6- Stellung des Pyridinkerns verknüpft sind.

**3.** 6-Chlor-4-hydroxy-2-methyl-N-[6-(2-benzo(b)furyl)-2-pyridyl]-2H-thieno[2,3-e]-1,2-thiazin-3-carbox-amid-1,1,-dioxid

**4.** 6-Chlor-4-hydroxy-2-methyl-N-[3-(2-phenylethyl)-2-pyridyl)]-2H-thieno[2,3-e]1,2-thiazin-3-carboxamid-1,1-dioxid

**5.** Verfahren zur Herstellung von Verbindungen der allgemeinen Formel (I) nach Anspruch 1, das dadurch gekennzeichnet ist, daß man eine Verbindung der allgemeinen Formel II

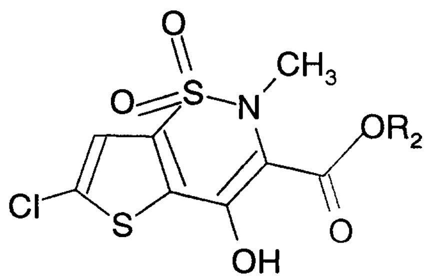

II

in der $R_2$ eine geradkettige oder verzweigte Alkylgruppe mit 1 - 4 C-Atomen bedeutet mit einer Verbindung der allgemeinen Formel III

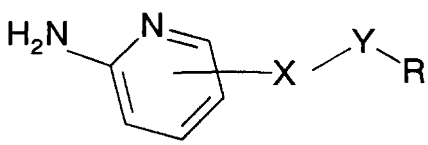

III

in der X, Y, R die oben genannte Bedeutung haben, umsetzt und die so erhaltenen Verbindungen der allgemeinen Formel (I) gegebenenfalls in ihre pharmazeutisch verwendbaren Salze überführt.

**6.** Verfahren nach Anspruch 5, dadurch gekennzeichnet, daß die Umsetzung in Gegenwart einer starken Base durchgeführt wird.

**7.** Pharmazeutische Präparate enthaltend Verbindungen der allgemeinen Formel (I) nach Anspruch 1, sowie deren Salze in Kombination mit üblichen galenischen Hilfs- und/oder Trägerstoffen.

**8.** Pharmazeutische Präparate nach Anspruch 7 in Kombination mit anderen therapeutisch wertvollen Verbindungen sowie Hilfs- und/oder Trägerstoffen.

**9.** Verbindungen nach Anspruch 1 zur Verwendung als Wirkstoffe für Arzneimittel zur Behandlung von Entzündungen und Schmerzzuständen.

**10.** Verbindungen nach Anspruch 1 als Antiinflammatorikum und Analgeticum.

Europäisches Patentamt

# EUROPÄISCHER RECHERCHENBERICHT

Nummer der Anmeldung

EP 93 10 9574

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | EP-A-0 001 113 (F. HOFFMANN-LA ROCHE)<br>* Ansprüche 1,19 *<br>--- | 1,7,10 | C07D513/04<br>A61K31/54<br>//(C07D513/04,<br>333:00,279:00) |
| A | EP-A-0 313 935 (CL PHARMA)<br>* Ansprüche 1,4,6 *<br>----- | 1,7,10 | |

| RECHERCHIERTE SACHGEBIETE (Int. Cl.5 ) |
|---|
| C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 27 SEPTEMBER 1993 | VOYIAZOGLOU D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)